# EUROPEAN PATENT SPECIFICATION

(11) **EP 1 688 730 B1**
(45) Date of publication and mention of the grant of the patent: **19.12.2012**
(21) Application number: 04792357.8
(22) Date of filing: 14.10.2004
(51) Int. Cl.: G01N 1/28

(54) **METHOD OF ANALYZING MINUTE QUANTITY OF CONTENT**
VERFAHREN ZUR ANALYSE KLEINSTER GEHALTSMENGEN
PROCEDE D'ANALYSE D'UNE QUANTITE INFIME D'UN CONTENU

(30) Priority: 26.11.2003 WO PCT/JP03/15090
(43) Date of publication of application: 09.08.2006
(73) Proprietor: MITSUBISHI DENKI KABUSHIKI KAISHA, Chiyoda-ku, Tokyo 100-8310 (JP)
(72) Inventor: NAKA, Jiro, c/o Mitsubishi Denki Kabushiki Kaisha, Tokyo, 1008310 (JP); KUROKAWA, Hiroshi, c/o Mitsubishi Denki K.K., Tokyo, 1008310 (JP); KOBAYASHI, Junji, c/o Mitsubishi Denki K.K., Tokyo, 1008310 (JP); TOYAMA, Satoru, c/o Mitsubishi Denki K.K., Tokyo, 1008310 (JP); HIRANO, Noriko, c/o Mitsubishi Denki K.K., Tokyo, 1008310 (JP); HARA, Eiji, c/o Mitsubishi Denki Kabushiki Kaisha, Tokyo, 1008 310 (JP)
(74) Representative: Popp, Eugen
(86) International application number: PCT/JP2004/015125
(87) International publication number: WO 2005/052552

(56) References cited:
- EP-A1- 0 601 689
- EP-A2- 0 672 831
- WO-A1-95/34360
- JP-A- 4 293 249
- JP-A- 6 174 616
- JP-A- 58 113 760
- JP-A- 2001 077 158
- JP-A- 2001 077 158
- JP-A- 2002 184 828
- JP-A- 2003 139 666
- US-A- 5 453 380
- US-A- 5 750 008
- US-A- 5 858 178
- US-A1- 2002 081 748

## Description

### TECHNICAL FIELD

The present invention relates to methods of analyzing minute quantities of contents in materials, specifically relates to methods of analyzing minute quantities of contents such as additives included in polymer materials.

### BACKGROUND ART

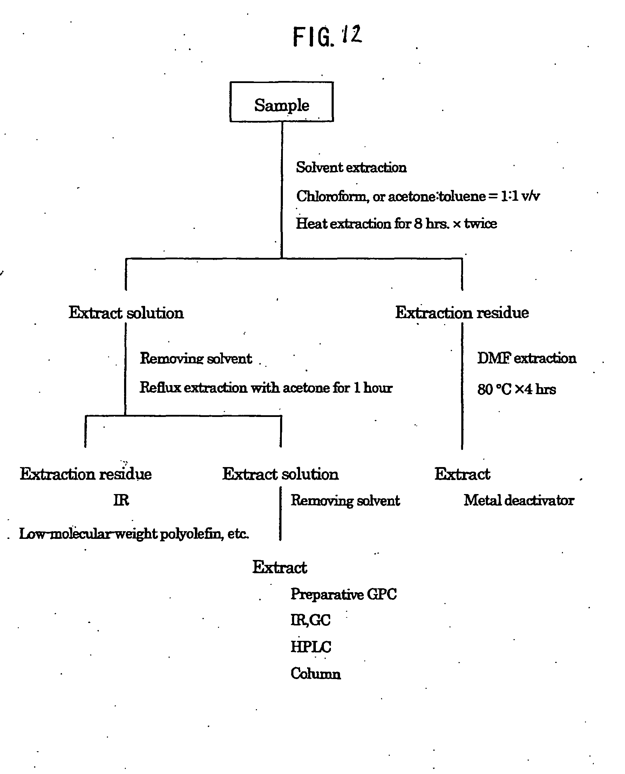

A flowchart is illustrated in Fig. 12 that represents a conventional method of analyzing additives included in polyolefin-group resin such as polypropylene (referred to as PP) and polyethylene (referred to as PE). First, the additives are extracted for 8 hours with a solvent such as chloroform heated up close to its boiling point, from pellets of the polyolefin-group resin as a sample (referred to as processing "A"). Here, this extraction is performed twice, and thus, all of the additives are extracted. Next, after chloroform is removed from the chloroform extract, the reflux extraction is performed for 1 hour using heated acetone (referred to as processing "B"); then, using this extract after acetone is removed, analysis is performed by either the liquid chromatography analyzer or the gas chromatography analyzer; consequently, the additives such as an antioxidant and a flame retardant are identified and quantified. On the other hand, regarding the residues remaining after the chloroform extraction, extraction is performed for 4 hours using heated N,N-dimethylformamide (referred to as processing "C"); then, the extract obtained is analyzed by the infrared spectrum analyzer, and thus, an additive such as a metal deactivator is identified.

In the processing "A", an acetone/toluene solvent mixture of 1:1 by volume ratio can also be used as the solvent other than chloroform. As a method for the processing "A", for example, the Soxhlet extraction method is used, in which this extraction is not limited to twice, but performed more than twice in response to necessity Here, in the Soxhlet extraction method used for the processing "A", because the extraction is performed with the solution being reflexed, a specified volume of the solution is needed; thus, as chloroform for example, the volume of approximately 100 ml is needed. Therefore, the amount of approximately 10 g is used for the sample pellets. Additionally, in the processing "A", because the extraction is performed using the solvent heated up close to its boiling point, due to the resin of the base material being partially extracted, this causes interference in the analysis; therefore, by re-extracting the chloroform extract using acetone that can only extract the additives, the resin component as the interference in the analysis is removed. Here, in the processing "A", if a solvent that extracts only the additives is used, the extraction time becomes further long (for example, referred to as Non-Patent Document 1).

### [Non-Patent Document 1]

Technical Information Institute, Ed., "Separation and Analysis Technology of Polymer Additives", on page 19 -21.

The most relevant prior art may be considered to be patent documents JP 2001/077158A, JP 58113760A, US 5858178A, EP 0672831 A2, and US 5750008A.

JP 2001/077158A discloses a method to improve the accuracy in analysing metallic contamination, such as platinum on a surface of a silicon wafer. A surface of a wafer is dipped in a solution of aqua regia diluted with pure water for a given time, and the diluted aqua regia is recollected. The aqua regia is vaporized to dried, solidified, and resolved in a nitric acid. Then, the metallic contamination on the surface of the silicon wafer is analyzed by inductively coupled plasma mass spectrometry or atom absorption spectrometry.

JP 58113760A discloses an injection dilution apparatus for testing samples. A sample is placed on a sample table of an injection and dilution vessel and the sample is kept in a fixed shape by its surface tension. A fixed flow rate is kept in an outflow passage in accordance with drive of a first pump and a fixed quantity of a buffer solution is drawn into an inflow passage by a second pump. Accordingly, the sample on the table is drawn into an inflow passage according to the difference between the suction quantity drawn by the first pump and that drawn by the second pump. Consequently, a diluted sample solution having an exact dilution ratio is obtained in the passage.

US 5858178A discloses a process for preparing and/or extracting samples by heating them together with a solvent in a container under pressure, using a Soxhlet apparatus. The samples are dried by heating in a container; and the resultant vapors are drawn off to generate a vacuum in the container.

EP 0672831 A2 discloses a pumping system for supercritical extraction. The system comprises an inlet connected to a source of supercritical fluid; an outlet to provide the pumped fluid to a pressure vessel; and a pumphead having a pumping chamber communicating with the inlet and outlet. The system further comprises a piston; an inlet valve controlling the flow of fluid into the pumping chamber through the inlet; an outlet valve controlling the flow of fluid from the pumping chamber through the outlet; an inlet conduit defining a flow path between the inlet valve and the pump chamber; and an outlet conduit defining a flow path between the pump chamber and the outlet valve. The system is characterized by an air-cooled thermoelectric-cooled heat exchanger for cooling both the inlet and the pumphead.

US 5750008A discloses a method for the extraction treatment of a sample with a solvent which is liquid at normal ambient temperature and normal pressure. According to the method, the solvent is introduced into a heatable sample chamber and brought into contact with the sample. The solvent vapour arising in the sample chamber is drawn off out of the sample chamber in such a manner that an under-pressure arises in the sample chamber and a further part of the liquid solvent passes into the vapour phase. The drawn off solvent vapour is cooled outside the sample chamber, and, for the purpose of recovery of pure solvent, is at least partially passed back into the liquid phase. The drawn-off solvent vapour is additionally subjected to over-pressure.

### DISCLOSURE OF THE INVENTION

As described above, in the conventional method of analyzing the minute quantity of the content, although the step of analyzing the extract-processed content has not been needed for a long time because of using instrumental analysis, regarding the step of preparing the sample, because not only a plural number of extraction treatment using same methods needed for a long time is performed, but also a plurality of different methods is also performed, it has been needed for a remarkably long time; consequently, a problem has occurred in which the minute quantity of the content cannot be rapidly identified and quantified.

An objective of the present invention, which is made to solve the above described problem, is to provide a method of rapidly analyzing a minute quantity of a content included in a material, in which sample preparation when the minute quantity of the content included in the material is analyzed is performed by once short time extraction treatment without a plural number of the extraction treatment taking along time and a plurality of different extraction-treatment methods.

According to a first aspect of the present invention, a method of analyzing a minutes quantity of content by analyzing extract extracted with a solvent from the content included in a polymer material includes a step of mounting, in contact with the top face comprising silver or gold substract of a sample table, a sample piece of the material to be analyzed; a step of dropping onto the sample table the solvent for extracting the content from the sample piece, and injecting the solvent into a gap between the top face of the sample table and the sample piece mounted in contact with the top face of the sample tabled; a step of maintaining at room temperature the solvent injected into the gap between the top face of the sample table and the sample piece, and, with the solvent maintained in the gap between the top face of the sample table and the sample piece, extracting the content from the sample piece; and a step of analyzing the content extracted from the sample piece using the time of slight secondary ion mass spectromety method.

According to a further aspect of the present invention, in the method of analyzing the minute quantity of the content according to the second aspect, the step of analyzing the content extracted from the sample piece includes a method of, after removing by vaporization of the solvent in the solution including the content extracted from the sample piece so as to deposit the content onto the surface of a substrate used as the sample table, analyzing the content deposited on the surface of the substrate.

According to another aspect of the present invention, in the method of analyzing the minute quantity of the content as the step of extracting the content from the sample piece, a method of extracting, by adding vibration in a state in which the solvent is maintained at room temperature in the gap between the top face of the sample table and the sample piece, using the solvent maintained in the gap between the top face of the sample table and the sample piece, the content from the sample piece is used.

According to another aspect of the present invention, in the method of analyzing the minute quantity of the content according to the second aspect, as the step of extracting the content from the sample piece, a method of extracting, by maintaining the solvent in the gap between the top face of the sample table and the sample piece in the saturated vapor atmosphere, at room temperature, of the solvent used for the extraction, using the solvent maintained in the gap between the top face of the sample table and the sample piece, the content from the sample piece is used.

According a still further aspect of the present invention, in the method of analyzing the minute quantity of the content the solvent, for extracting the content from the sample piece additionally includes a silver composition soluble in the solvent.

This allows the extraction time to be shortened, and, using a small amount of the sample piece, accurate analysis of the content in the material can be performed in a short time.

Furthermore, the sensitivity, using the time of flight secondary ion mass spectrometry method, for analyzing the extract from the material is remarkably improved.

### BRIEF DESCRIPTION OF DRAWINGS

Fig. 1 is a flow chart explaining a method of analyzing a minute quantity of a content included in a material according to the present invention;
Fig. 2 is views illustrating states in which an extraction solvent is dropped, according to the analyzing method of the present invention;
Fig. 3 is a view illustrating a state, according to the analyzing method of the present invention, in which a sample piece is mounted in contact with the top face of a sample table, and the extraction solvent is maintained in gaps between the top face of the sample table and the sample piece;
Fig. 4 is views illustrating a first method of preparing a specimen, for analyzing extract by an analyzer, according to an analyzing method of the present invention;
Fig. 5 is, as an example of the measurement results according to Example 1, a mass spectrum of extract extracted from a PP pellet including the brominated flame retardant of 100 ppm;
Fig. 6 is a graph representing a relationship between the mass-spectrum peak-area ratios (⁷⁹Br/¹⁰⁷Ag) obtained from the analysis in which the extracts are extracted from the PP pellets each including the brominated flame retardant of 1 ppm, 10 ppm, 100 ppm, 1000 ppm, 1%, or 10% as the concentration, and the brominated flame retardant concentrations, according to Example 1;.
Fig. 7 is, as an example of the measurement results according to Example a mass spectrum of extract extracted from an HIPS pellet including the brominated flame retardant of 0.1%;
Fig. 8 is a graph representing a relationship between the mass-spectrum peak-area ratios (1068(B+Ag)⁺/107Ag⁺) obtained from the analysis in which the extracts are extracted from the HIPS pellets each including the brominated flame retardant of 0.1%, 1%, or 10% as the concentration, and the brominated flame retardant concentrations, according to Example 2;
Fig. 9 is a view representing a state According to Example 3, in which a content is extracted from a sample piece;
Fig. 10 is a mass spectrum of the extract, obtained by the method according to Example 3, extracted from the PP pellet including the brominated flame retardant of 100 ppm;
Fig. 11 is a mass spectrum of extract, obtained by a method according to Example 4, extracted from the HIPS pellet including the brominated flame retardant of 0.1%; and of 0.1%; and
Fig. 12 is a flowchart representing a conventional method of analyzing an additive included in polyolefin-group resin.

### BEST MODE FOR CARRYING OUT THE INVENTION

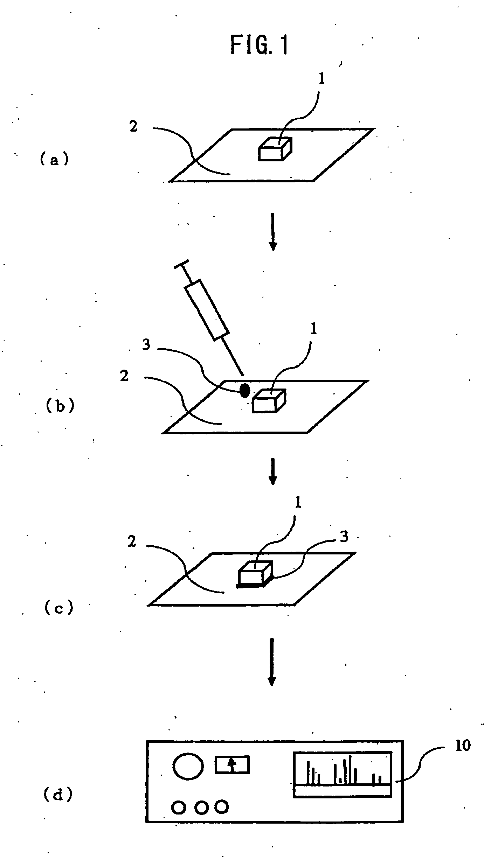

Fig. 1 is a flow chart explaining a method of analyzing a minute quantity of a content included in a material according to the present invention. In a first step, a sample piece 1 of the material including a substance to be analyzed is mounted in contact with the top face of a sample table 2 (Fig. 1(a)). In a second step, a solvent 3 for extracting the content from the sample piece 1 is dropped onto the top face of the sample table 2 so as to inject the solvent into the gaps between the top face of the sample table 2 and the sample piece 1 (hereinafter referred to as "gaps between then sample table 2 and the sample piece 1") (Fig. 1(b)). In a third step, the solvent 3 injected into the gaps between the sample table 2 and the sample piece 1 is kept for a short time at room temperature; thus, by the solvent 3 maintained in the gaps between the sample table 2 and the sample piece 1, the content to be analyzed is extracted from the sample piece 1 (Fig. 1(c)). In a forth step, the content extracted from the sample piece 1 is analyzed by an instrumental analyzer 10 (Fig. 1(d)).

In the analyzing method according to the present invention, as the material to be analyzed, polymer materials such as plastic, rubber, adhesives, encapsulating resin, and mold resin are listed. These polymer materials are analyzed not only in the state of the materials themselves, but also in a state in which the materials are used in instrumental parts such as an instrumental case, a molded product, and a printed wiring board. In the analyzing method according to the present invention, as materials to be analyzed, a sub-material such as an antioxidant, a fire retardant, a curing catalyst, or a processing aid included in a polymer material, as well as a minute quantity of a substance that may be included either during the material itself being produce, or during the material being molded/processed into various parts of a product can be listed; however, if the substance that can be extracted with a solvent from the polymer material to be applied is used, the material is not limited, to the above. In the analyzing method according to the present invention, a little amount of the sample piece such as an approximately one resin-pellet amount (for example, 0.1 - 0.5 g in weight) may also be used.

In the analyzing method according to the present invention, as the sample table for mounting the sample piece, any table having a flat face that can mount the sample piece may be applied, and especially, a substrate is preferably applied. When the time-of-flight secondary ion mass spectrometry method is applied as the analyzing method, for example, a silver substrate, a gold substrate, or an SUS substrate on which silver or gold is plated is used.

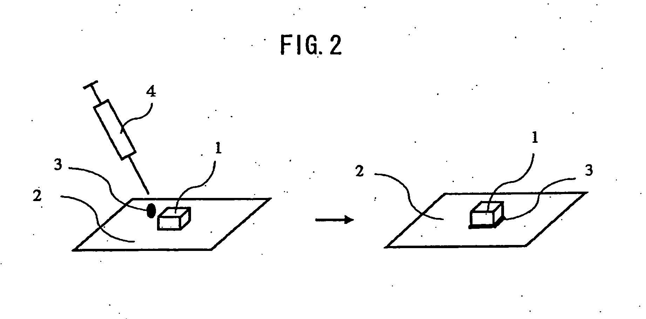

Fig. 2 is views illustrating states in which the extraction solvent is dropped, according to the analyzing method of the present invention. As represented in Fig. 2, the extraction solvent 3 is dropped using a microsyringe 4 onto the top face of the sample table 2 with which the pellet of the sample piece 1 is mounted in contact. In Fig. 2, a substrate as the sample tables 2 is represented as an example; hereinafter, a substrate 2 is explained as the sample table 2. However, according to the present invention, the sample table 2 is not limited to the substrate. Regarding the dropping volume of the extraction solvent 3, the volume may be from a volume that can at least fill the gaps between the substrate 2 and the sample piece 1 to a volume that is twice the volume of the sample piece; thereby, for example, when the sample piece 1 is a single resin-pellet, the volume is 5 - 100 µl Moreover, if the position to be dropped is on the top face of the substrate 2, the position is not especially limited; however, it is preferable to drop the solvent at a position, on the top face of the substrate 2, close to a portion on which the sample piece 1 is mounted, specifically, to drop at the boundary between the portion on which the sample piece 1 is mounted and the portion on which the sample piece 1 is not mounted, because the solvent 3 can be effectively injected into the gaps between the substrate 2 and the sample piece 1.

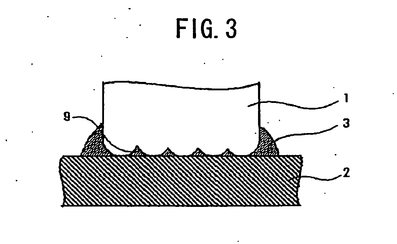

Fig. 3 is a view illustrating a state in which the sample piece 1 is mounted in contact with the top face of the substrate as the sample table 2. As represented in Fig. 3, the sample piece 1 has recesses and protrusions on its face contacting to the substrate 2; thereby, these protrusions contact to the top face of the substrate 2, meanwhile these recesses form gaps 9 between the substrate 2 and the sample piece 2, and thus, the solvent dropped is injected into the gaps 9. In the extraction of the content from the sample piece 1 according to the analyzing method of the present invention, the state in which the solvent 3 is at least maintained in the gaps 9 between the substrate 2 and the sample piece 1 is held at room temperature for a short time; thereby, the content is extracted into the solvent 3 contacting to the sample piece, especially into the solvent 3 existing in the gaps between the substrate 2 and the sample piece 1. At this time, because the solvent decreases due to its vaporization, after a predetermined time passes, the solvent 3 may be dropped again. For example, if the sample piece 1 is the single resin-pellet, the extraction time, that is, the time during the state in which the solvent 3 is maintained in the gaps between the substrate 2 and the sample piece 1 being held, and the content being extracted is preferably set for 0.5 - 30 minutes, and further preferably set for 0.5 - 15 minutes. If this time is shorter than 0.5 minutes, the extraction becomes insufficient; thereby, the analysis accuracy deteriorates. On the other hand, if the time is longer than 30 minutes, the repeating number of the dropping only increases without increase of the extract amount; thereby, not only the analysis process becomes more complex, but also the analysis time becomes longer.

Moreover, in order to increase the amount of the content extracted from the sample piece 1 into the solvent 3, the substrate 2 may be vibrated during the extraction. As the vibration method, a method of using an ultrasonic washer or a shaker, and a method in which an ultrasonic oscillator is pasted onto the substrate 2 are listed. Furthermore, by putting into a sealed container the substrate 2, the sample piece 1, and the solvent 3 maintained in the gaps between the substrate 2 and the sample piece 1, the extraction of the content may be performed from the sample piece 1 using the extraction solvent 3, in the saturated vapor atmosphere of the same solvent as the extraction solvent 3. According to this operation, loss of the extraction solvent 3 due to the vaporization is prevented, and additional dropping of the solvent becomes needless; consequently, the analyzing process can be simplified.

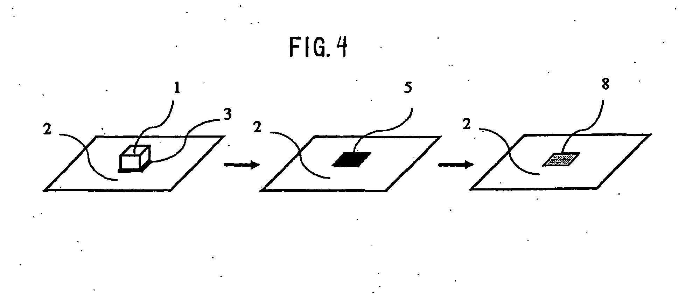

Fig. 4 is views illustrating a first method of preparing a specimen, for analyzing the extract by an analyzer, according to an analyzing method of the present invention. This second method is used when the extract is analyzed by the time-of-flight secondary ion mass spectrometry method. As represented in Fig. 4, after the extraction step has finished, the test piece 1 is removed from the substrate 2, and then, the solvent of the solution 5 including the extract placed on the top face of the substrate 2 is removed by vaporization; thus, the substrate surface on which extract 8 is deposited is directly analyzed by the analyzer. In the analyzing method of the present invention, the extract is analyzed using the time-of-flight secondary ion mass spectrometry method, because if the extract exists too much, the deposition portion is charged up; therefore, in order to prevent the charging up, it is preferable that a silver substrate, a gold substrate, or an SUS substrate on which silver or gold is plated is used as the substrate. In the analyzing method of the present invention, as the solvent used for extracting, a solvent is used that extracts the content without decomposing the polymer material at room temperature. Regarding the grade of the solvent used, a solvent having the analysis grade purity is preferably used because of little influence on analyzing the content.

In the analyzing method of the present invention, the extract is analyzed using the time-of-night secondary ion mass spectrometry method, the content is dissolved in a solvent for extraction, and, if the solution is used in which a silver compound that does not include as an impurity the substance to be measured is added, not only the charging up can be prevented even if a chargeable substrate is used, but also the analysis sensitivity is improved; consequently, the analysis accuracy is improved. In the analyzing method of the present invention, the sample piece is mounted in contact with the top face of the sample table such as the substrate, the solvent is injected by dropping into the gaps between the sample table and the sample piece, the solvent injected is maintained in the gaps between the sample table and the sample piece, the content is extracted with this maintained solvent, and the extract is analyzed by the analyzer; therefore, the extraction time can be shortened, and using a small amount of the sample piece, accurate analysis of the content in the material, especially in the polymer material, can be performed in a short time. Hereinafter, more specific examples according to the present invention are represented; however, the present invention is not limited to these examples.

### EXAMPLES

### Example 1.

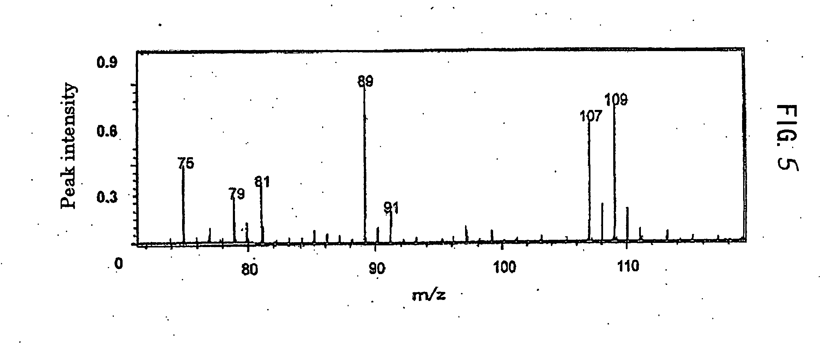

PP pellets each including the brominated flame retardant of 1 ppm, 10 ppm, 100 ppm, 1000 ppm, 1%, or 10% by weight concentration were prepared as the sample pieces 1. Next, a silver substrate was used as a substrate for the sample table 2, and extract from each PP pellet was deposited as a condensed substance on the surface of the substrate. In this example, the deposited substance on the surface of the substrate was analyzed by the time-of-flight secondary ion mass spectrometry method. TRIFT2™ (manufactured by ULVAC-PHI Inc.) was used as the time-of-flight secondary ion mass spectrometry analyzer. Regarding the measurement condition, ⁶⁹Ga⁺ ion was used as the primary ion, the measurement mode of the secondary ion was set to the negative ion mode, the measurement range was set to m/z = 1 - 200, and the mass resolution was set to approximately ΔM/M = 5000. Fig. 5 is, as an example of the measurement results, a mass spectrum of the extract extracted from the PP pellet including the brominated flame retardant of 100 ppm. As represented in Fig. 14, the mass-spectrum peak caused by the fragment of the bromine element was observed at m/z = 79. Quantitative analysis was performed using the normalized (79Br⁻/¹⁰⁷Ag⁻) peak-area ratio in which the area of the peak at m/z = 79 (⁷⁹Br⁻) is normalized by the area of the peak at m/z = 107 (¹⁰⁷Ag⁻) caused by the fragment of silver in the substrate.

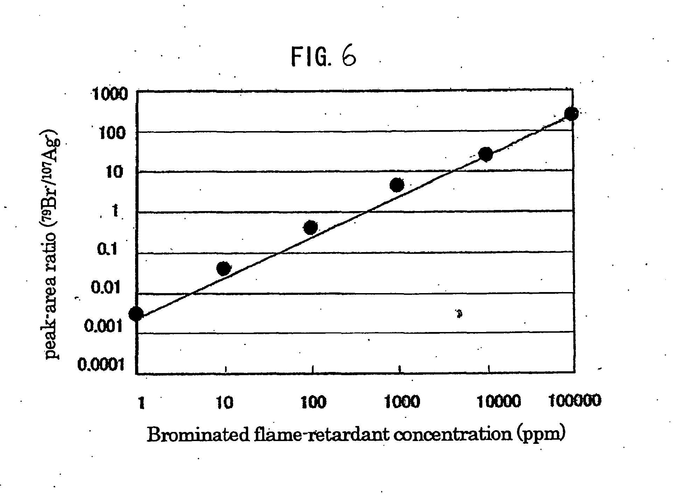

Fig. 6 is a graph representing a relationship between the mass-spectrum peak-area ratios (⁷⁹Br⁻/¹⁰⁷Ag⁻) obtained from the analysis of the extracts extracted from the PP pellets each including the brominated flame retardant of 1 ppm, 10 ppm, 100 ppm, 1000 ppm, 1%, or 10% as the concentration, and the brominated flame retardant concentrations. An excellent linear relationship was obtained between the brominated flame retardant concentrations and the peak-area ratios, and especially, it was found to be also detectable at the minute concentration of 1 ppm. In this example, the processing time was 10 minutes for extracting the brominated flame retardant as the content from the PP pellet of the sample piece 1; thereby, it was found that the quantitative analysis, also up to such minute concentration, of the brominated flame retardant as the content can be performed by a short-time extraction operation. As described above, in the analyzing method According to this example, the extraction processing time can also be considerably shortened compared to that in the conventional method, and the brominated flame retardant minutely included, for example, 1 ppm, in the PP specimen can be rapidly analyzed.

### Example 2.

High impact polystyrene (referred to as HIPS) specimens each including brominated flame retardant as the additive of 0.1%, 1%, or 10% by weight were prepared as the samples. H8672™ (produced by PS Japan Corp.) was used as the HIPS, and decabromodiphenylether (produced by Wako Pure Chemical Industries, Ltd.) was used as the brominated flame retardant. As the sample piece 1, the brominated flame retardant was added to and kneaded with the HIPS so that the concentration becomes above each value; thus, pellets were prepared in which the size of the single pellet is 5 mm x 3 mm x 3mm, and the weight is approximately 0.3 g. Similarly to the procedure represented in Fig. 2, the single HIPS pellet as the sample piece 1 was mounted in contact with a silver substrate as the sample table 2, a mixed solvent of toluene and methanol (toluene/methanol =1/1 by volume) as the extraction solvent 3 of 20 µl was dropped, using the microcyringe 4, close to the HIPS pellet, so as to inject the mixed solvent into the gaps between the HIPS pellet and the silver substrate, and then, the sample piece was kept. This mixed solvent is a solvent for extracting not only the HIPS but also the brominated flame retardant. Then, after 30 seconds passed from the drop operation, the HIPS pellet was removed from the silver substrate, nitrogen gas was blown onto the surface of the silver substrate on which the HIPS pellet was removed, and the solvent containing the brominated flame retardant was dried; thus, extract was deposited on the surface of the silver substrate. The processing time was approximately one minute from this mixed solvent being dropped until the extract was deposited onto the surface of the silver substrate. The grades of toluene and methanol used in this example were the liquid chromatographic ones (produced by Wako Pure Chemical Industries, Ltd.).

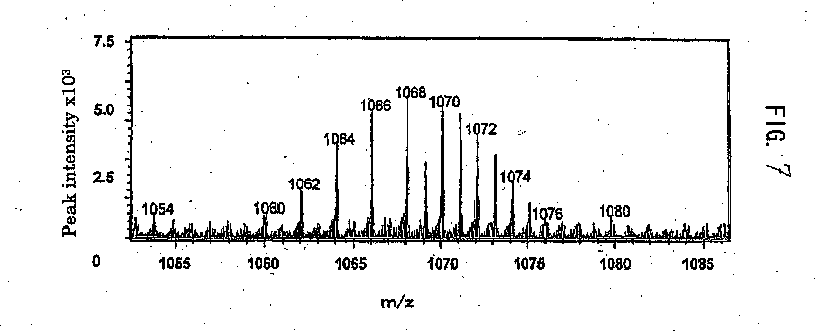

In this example, the deposited substance on the surface of the substrate was analyzed by the time-of flight secondary ion mass spectrometry method. TRIFT2™ (manufactured by ULVAC-PHI Inc.) was used as the time-of-flight secondary ion mass spectrometry analyzer. Regarding the measurement condition, ⁶⁹Ga⁺ ion was used as the primary ion, the measurement mode of the secondary ion was set to the positive ion mode, the measurement range was set to m/z = 1 - 1500, and the mass resolution was set to approximately ΔM/M = 5000. Fig. 7 is, as an example of the measurement results, a mass spectrum of the extract extracted from the HIPS pellet including the brominated flame retardant of 0.1%. As represented in Fig. 16, the mass-spectrum peak caused by the peak B⁺ due to the fragment of decabromodiphenylether as the brominated flame retardant and the peak Ag⁺ due to the fragment of silver was observed at m/z = 1068. Quantitative analysis was performed using the normalized (¹⁰⁶⁸(B+Ag)+/¹⁰⁷Ag⁺) peak-area ratio in which the area of the peak at m/z = 1068 (¹⁰⁶⁸(B+Ag)⁺) is normalized by the area of the peak at m/z. = 107 (¹⁰⁷Ag⁺). The above area ratio of the extract extracted from the HIPS pellet including the brominated flame retardant of 0.1% was 0.005.

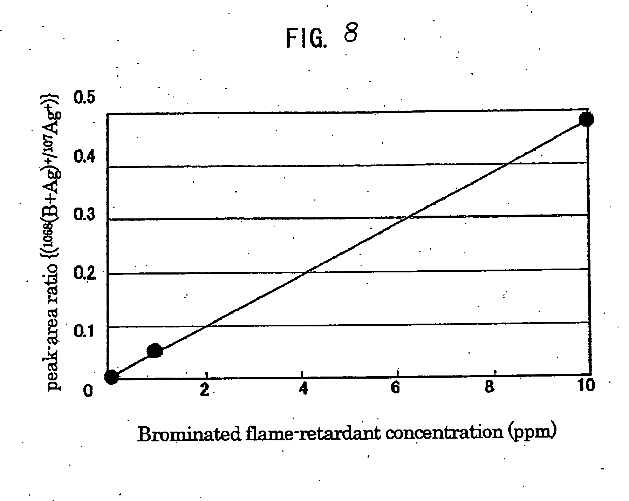

Fig. 8 is a graph representing a relationship between the mass-spectrum peak-area ratios (¹⁰⁶⁸(B+Ag)^{+/107}Ag⁺) obtained from the analysis of the extracts being extracted from the HIPS pellets each including the brominated flame retardant of 0.1%, 1%, or 10% as the concentration, and the brominated flame retardant concentrations. An excellent linear relationship was obtained between the brominated flame retardant concentrations and the peak-area ratios. In this example, the processing time was 1 minute for extracting the brominated flame retardant as the content from the HIPS pellet; thus, it was determined that the quantitative analysis, up to also the minute concentration, of the brominated flame retardant as the content can be performed by an extremely short-time extraction operation. As described above, in the analyzing method according to this example, the extraction processing time can be considerably shortened compared to that in the conventional method, and a content included in a matrix that is soluble in a solvent extracting the content, such as the brominated flame retardant included in the HIPS specimen, can also be rapidly analyzed. further minute, the content can also be accurately analyzed in a short time.

### Embodiment 3.

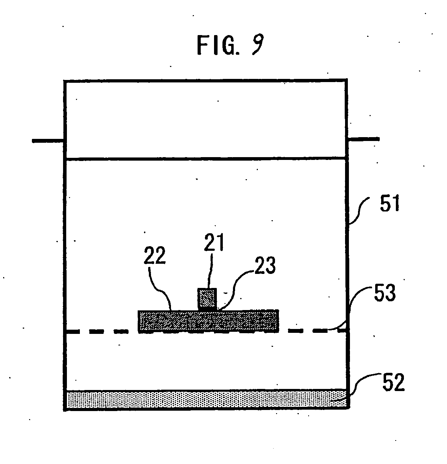

In this example, similarly to the procedure in Example 1, a PP pellet as the sample piece 1 including the brominated flame retardant of 100 ppm by weight was prepared. This pellet was mounted in contact with a silver substrate as the sample table 2; then, similarly to the procedure in Example 1, after toluene as the extraction, solvent 3 was dropped and injected into the gaps between the PP pellet and the silver substrate, the sample piece was held. The sample piece was kept for 10 minutes in a state in which the toluene is maintained in the gaps between the PP pellet and the silver substrate; thereby, the brominated flame retardant was extracted into the toluene, so that the brominated flame retardant as the deposited substance was deposited on to the silver substrate as a condensed substance. Fig 9 is a view representing a state according to this example, in which the content is extracted from the sample piece. As represented in Fig. 9, during the extraction operation, a silver substrate 22, on which a PP pellet 21 is mounted, and between which and the PP pellet 21 toluene 23 is maintained in the gaps, is placed inside a sealed container 51 in which toluene vapor is saturated. Specifically, toluene 52 that generates its vapor is contained at the bottom of this sealed container 51, and a shelf plate 53 having holes is provided on the upper side of the toluene 52 that generates its vapor. The silver substrate 22 is placed on the top face of this shelf plate 53, the PP pellet 21 is mounted in contact with the top face of this silver substrate 22, and the toluene 23 is maintained in the gaps between the top face of the silver substrate 22 and the PP pellet 21. That is, because the PP pellet 21 is stored in saturated vapor of the toluene during the operation in which the brominated flame retardant is extracted from the PP pellet 21, loss, due to vaporization, of the toluene 23 as the extraction solvent can be prevented; therefore, the re-dropping of the toluene 23 becomes unnecessary, and the analysis process becomes simple. After the extraction, the silver substrate was taken out from the sealed container 51, the PP pellet 21 was removed from the silver substrate 22, and the solvent was dried with nitrogen gas being blown onto the surface of the silver substrate 22, so that the brominated flame retardant was deposited on the surface of the silver substrate 22 as a condensed substance.

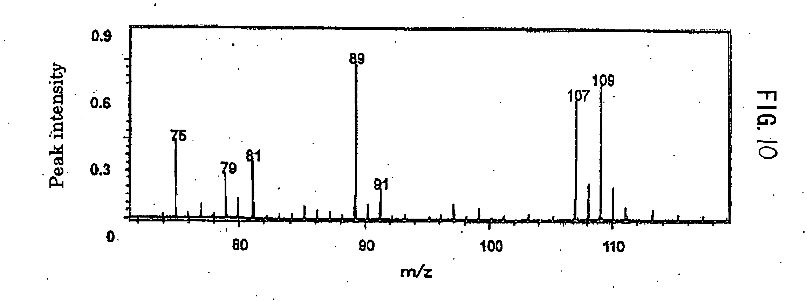

In this example, similarly to the method in Example 1, the deposited substance on the surface of the substrate was analyzed by the time-of flight secondary ion mass spectrometry method. Fig. 10 is a mass spectrum of the extract, obtained by the method according to this example, extracted from the PP pellet including the brominated flame retardant of 100 ppm. As represented in Fig. 10, a mass spectrum peak due to the fragment of the bromine element was observed at m/z = 79. Quantitative analysis of the brominated flame retardant included in the PP pellet could be performed using the normalized (⁷⁹Br⁻/¹⁰⁷Ag⁻) peak area ratio that is obtained from the area of the peak at m/z = 79 (⁷⁹Br⁻) being normalized by the area of the peak at m/z =107 (¹⁰⁷Ag) caused by the fragment of silver in the substrate. That is, in the method According to this example, not only the extraction processing time can be considerably shortened compared to that in the conventional method, but also the re-dropping of the extraction solvent becomes unnecessary; moreover, because of the simple process, the brominated flame retardant as the content included in the PP specimen can be rapidly analyzed.

### Example 4:

In this example, similarly to the procedure in Example 2, an HIPS pellet including the brominated flame retardant of 0.1% by weight was prepared. In this example, except for a mixed solvent of toluene and methanol (toluene/methanol = 1/1 by volume) in which silver perchlorate is saturated being used as the extraction solvent 3, similarly to the procedure in Example 2, extract from the HIPS pellet was deposited as a condensed substance on the surface of the silver substrate.

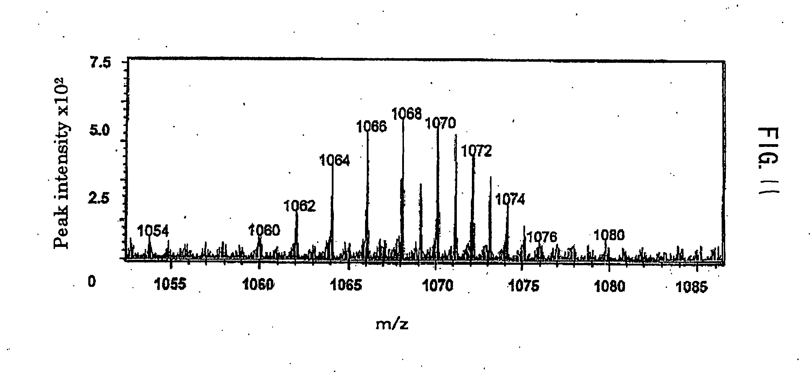

In this example, similarly to the method in Example 2, the deposited substance on the surface of the substrate was analyzed by the time-of-flight secondary ' ion mass spectrometry method. Fig. 11 is a mass spectrum of the extract, obtained by the method according to this example, extracted from the HIPS pellet including the brominated flame retardant of 0.1%. As represented in Fig. 11, the mass spectrum peak due to the fragments of decabromodiphenylether as brominated flame retardant and silver was observed at m/z = 1068. The normalized (¹⁰⁶⁸(B+Ag)^{+/107}Ag⁺) peak area ratio in which the area of the peak at m/z = 1068 (¹⁰⁶⁸(B+Ag)⁺) is normalized by the area of the peak at m/z =107 (¹⁰⁷Ag⁺) caused by the fragment of silver in the substrate was 0.05, which is ten times larger than that of Example 2 in which silver perchlorate as a conductive substance is not added. That is, in the method according to this example, compared to the conventional method, not only the extraction processing time can be considerably shortened, but also the sensitivity for analyzing the extract is remarkably improved; consequently, the brominated flame retardant as the content included in the HIPS specimen can be rapidly analyzed.

### INDUSTRIAL APPLICABILITY

The method of analyzing the minute quantity of the content according to the present invention can be used for analyzing a minute quantity of a content such as an addictive included in a polymer material such as plastic, rubber, adhesives, encapsulating resin, or mold resin. Moreover, a minute quantity of a content included in a polymer material constituting an instrumental part such as a case, a molded product, and a printed wiring board that are manufactured using the polymer material can be analyzed.

## Claims

1. A method of analyzing a minute quantity of content by analyzing the content extracted with a solvent (3) from a polymer material including the content, the method comprising:
a step of mounting, in contact with the top face of a silver substrate, a gold substrate, or an SUS substrate on which silver or gold is plated, used as the sample table (2), a sample piece (1) of the material to be analyzed;
a step of dropping, onto the sample table (2), the solvent (3) for extracting the content from the sample piece (1), and injecting the solvent (3) into a gap (9) between the top face of the sample table (2) and the sample piece (1) mounted in contact with the top face of the sample table (2);
a step of maintaining at room temperature the solvent (3) injected into the gap (9) between the top face of the sample table (2) and the sample piece (1), and, with the solvent (3) maintained in the gap (9) between the top face of the sample table (2) and the sample piece (1), extracting the content from the sample piece (1); and
a step of removing the sample piece (1) from the sample table (2), vaporizing the solvent (3) remaining on the top face of the sample table (2), and analyzing a surface of the sample table (2) after the vaporization using the time-of-flight secondary ion mass spectrometry method.

2. A method of analyzing a minute quantity of content as recited in claim 1, wherein the solvent (3) for extracting the content includes a silver composition that is dissolved therein.

3. A method of analyzing a minute quantity of content as recited in claim 1 or 2, wherein, in the step of extracting the content from the sample piece (1), a method of extracting, by adding vibration in a state in which the solvent (3) is maintained at room temperature in the gap (9) between the top face of the sample table (2) and the sample piece (1), using the solvent (3) maintained in the gap (9) between the top face of the sample table (2) and the sample piece (1), the content from the sample piece (1) is used.

4. A method of analyzing a minute quantity of content as recited in claim 1 or 2, wherein, in the step of extracting the content from the sample piece (1), a method of extracting, by maintaining the solvent (3) in the gap (9) between the top face of the sample table (2) and the sample piece (1) in the saturated vapor atmosphere, at room temperature, of the solvent (3) used for the extraction, using the solvent (3) maintained in the gap (9) between the top face of the sample table (2) and the sample piece (1), the content from the sample piece (1) is used.

## Patentansprüche

1. Verfahren zum Analysieren einer kleinsten Inhaltsmenge, indem der Inhalt analysiert wird, der mit einem Lösungsmittel (3) aus einem den Inhalt enthaltenden Polymermaterial extrahiert wird, wobei das Verfahren umfasst:
einen Schritt des Anbringens, und zwar in Kontakt mit der Oberseite eines als Probentisch (2) verwendeten Silbersubstrats, Goldsubstrats oder SUS-Substrats, auf das Silber oder Gold aufgetragen ist, eines Probenstücks (1) des zu analysierenden Materials;
einen Schritt des Auftropfens des Lösungsmittels (3) auf den Probentisch (2), um den Inhalt aus dem Probenstück (1) zu extrahieren, und des Einspritzens des Lösungsmittels (3) in einen Spalt (9) zwischen der Oberseite des Probentischs (2) und dem in Kontakt mit der Oberseite des Probentischs (2) angebrachten Probenstück (1);
einen Schritt des Haltens des in den Spalt (9) zwischen der Oberseite des Probentischs (2) und dem Probenstück (1) eingespritzten Lösungsmittels (3) bei Raumtemperatur, und, wobei das Lösungsmittel (3) in dem Spalt (9) zwischen der Oberseite des Probentischs (2) und dem Probenstück (1) gehalten wird, des Extrahierens des Inhalts aus dem Probenstück (1); und
einen Schritt des Entfernens des Probenstücks (1) vom Probentisch (2), des Verdampfens des auf der Oberseite des Probentischs (2) verbliebenen Lösungsmittels (3), und des Analysierens einer Oberfläche des Probentischs (2) nach dem Verdampfen unter Nutzung des Flugzeit-Sekundärionenmassenspektrometrieverfahrens.

2. Verfahren nach Anspruch 1 zum Analysieren einer kleinsten Inhaltsmenge, wobei das Lösungsmittel (3) zum Extrahieren des Inhalts eine Silberzusammensetzung umfasst, die darin gelöst ist.

3. Verfahren nach Anspruch 1 oder 2 zum Analysieren einer kleinsten Inhaltsmenge, wobei im Schritt des Extrahierens des Lösungsmittels aus dem Probenstück (1), ein Schritt des Extrahierens des Inhalts aus dem Probenstück (1) eingesetzt wird, indem in einem Zustand, in dem das Lösungsmittel (3) bei Raumtemperatur in dem Spalt (9) zwischen der Oberseite des Probentischs (2) und dem Probenstück (1) gehalten wird, eine Schwingung angelegt wird, unter Verwendung des Lösungsmittels (3), das im Spalt (9) zwischen der Oberseite des Probentischs (2) und dem Probenstück (1) gehalten wird.

4. Verfahren nach Anspruch 1 oder 2 zum Analysieren einer kleinsten Inhaltsmenge, wobei im Schritt des Extrahierens des Lösungsmittels aus dem Probenstück (1), ein Schritt des Extrahierens des Inhalts aus dem Probenstück (1) eingesetzt wird, indem das Lösungsmittel (3) in dem Spalt (9) zwischen der Oberseite des Probentischs (2) und dem Probenstück (1) in der bei Raumtemperatur gesättigten Dampfatmosphäre des zur Extraktion verwendeten Lösungsmittels (3) gehalten wird, unter Verwendung des Lösungsmittels (3), das in dem Spalt (9) zwischen der Oberseite des Probentischs (2) und dem Probenstück (1) gehalten wird.

## Revendications

1. Procédé destiné à analyser une quantité infime de contenu en analysant au moyen d'un solvant (3) le contenu extrait d'un matériau polymère qui comprend le contenu, le procédé comprenant :
une étape consistant à monter un élément d'échantillon (1) du matériau à analyser en contact avec la face supérieure d'un substrat en argent, d'un substrat en or ou d'un substrat SUS sur lequel est plaqué de l'argent ou de l'or, utilisé en tant que table d'échantillon (2) ;
une étape consistant à déposer le solvant (3) sur la table d'échantillon (2) afin d'extraire le contenu de l'élément d'échantillon (1) et d'injecter le solvant (3) dans un interstice (9) entre la face supérieure de la table d'échantillon (2) et l'élément d'échantillon (1) monté en contact avec la face supérieure de la table d'échantillon (2) ;
une étape consistant à maintenir à température ambiante le solvant (3) injecté dans l'interstice (9) entre la face supérieure de la table d'échantillon (2) et l'élément d'échantillon (1), et, le solvant (3) étant maintenu dans l'interstice (9) entre la face supérieure de la table d'échantillon (2) et l'élément d'échantillon (1), à extraire le contenu de l'élément d'échantillon (1) ; et
une étape consistant à retirer l'élément d'échantillon (1) de la table d'échantillon (2), à vaporiser le solvant (3) restant sur la face supérieure de la table d'échantillon (2), et à analyser une surface de la table d'échantillon (2) après la vaporisation à l'aide d'un procédé de spectrométrie de masse des ions secondaires en temps de vol.

2. Procédé destiné à analyser une quantité infime de contenu selon la revendication 1, dans lequel le solvant (3) destiné à extraire le contenu comprend une composition d'argent qui est dissoute à l'intérieur.

3. Procédé destiné à analyser une quantité infime de contenu selon la revendication 1 ou 2, dans lequel, dans l'étape consistant à extraire le contenu de l'élément d'échantillon (1), on utilise un procédé consistant à extraire le contenu de l'élément d'échantillon (1) en ajoutant des vibrations dans un état où le solvant (3) est maintenu à température ambiante dans l'interstice (9) entre la face supérieure de la table d'échantillon (2) et l'élément d'échantillon (1), le solvant (3) étant maintenu dans l'interstice (9) entre la face supérieure de la table d'échantillon (2) et l'élément d'échantillon (1).

4. Procédé destiné à analyser une quantité infime de contenu selon la revendication 1 ou 2, dans lequel, dans l'étape consistant à extraire le contenu de l'élément d'échantillon (1), on utilise un procédé consistant à extraire le contenu de l'élément d'échantillon (1) en maintenant le solvant (3) dans l'interstice (9) entre la face supérieure de la table d'échantillon (2) et l'élément d'échantillon (1) dans l'atmosphère de vapeur saturée, à température ambiante, du solvant (3) utilisé pour l'extraction, le solvant (3) étant maintenu dans l'interstice (9) entre la face supérieure de la table d'échantillon (2) et l'élément d'échantillon (1).
